# EUROPÄISCHE PATENTSCHRIFT

(11) **EP 2 560 756 B1**
(45) Veröffentlichungstag und Bekanntmachung des Hinweises auf die Patenterteilung: **02.10.2019**
(21) Anmeldenummer: 11711574.1
(22) Anmeldetag: 01.04.2011
(51) Int. Cl.: B01L 3/00, G01N 33/49, G01N 1/28, B29C 65/54

(54) **VORRICHTUNG ZUR PLASMASEPARATION MITTELS EINER ZENTRALEN KANALSTRUKTUR**
DEVICE FOR PLASMA SEPARATION BY MEANS OF A CENTRAL CHANNEL STRUCTURE
DISPOSITIF POUR LA SÉPARATION PLASMATIQUE AU MOYEN D'UNE STRUCTURE DE CANAL CENTRALE

(30) Priorität: 23.04.2010 EP 10160817
(43) Veröffentlichungstag der Anmeldung: 27.02.2013
(73) Patentinhaber: Boehringer Ingelheim Microparts GmbH, 44227 Dortmund (DE)
(72) Erfinder: BLANKENSTEIN, Gert, 55216 Ingelheim am Rhein (DE); HELLMICH-DUONG, Thanh Tu, 55216 Ingelheim am Rhein (DE); KUROWSKI, Dirk, 55216 Ingelheim am Rhein (DE); OSTERLOH, Dirk, 55216 Ingelheim am Rhein (DE)
(74) Vertreter: Simon, Elke Anna Maria
(86) Internationale Anmeldenummer: PCT/EP2011/055078
(87) Internationale Veröffentlichungsnummer: WO 2011/131471

(56) Entgegenhaltungen:
- EP-A1- 0 597 577
- EP-A2- 1 013 341
- WO-A1-2009/075709
- WO-A1-2009/112982
- US-A- 4 906 439
- US-A1- 2007 269 893
- US-A1- 2009 120 865

## Beschreibung

Die Erfindung betrifft eine Vorrichtung zur Filterung eines Fluides und zum Ableiten des gefilterten Fluides, insbesondere Plasmas in eine diagnostische Testcartridge.

Stand der Technik wird gebildet durch Filtrationsgeräte für die Separation von Blutplasma. So zeigt die US 2009/0120865 A1 ein entsprechendes mehrlagiges Einweggerät, das an einen Biochip angesetzt wird. Das Filtrationsgerät umfasst aufeinandergestapelt und miteinander verbunden ein oberes Substrat mit einem Bluteinlass, ein mittleres Substrat mit der Filtereinheit zur Trennung des Plasmas vom Blut und ein unteres Substrat mit einem Luftauslass. In der Filtereinheit liegt (in einer Filterkammer) der eigentliche Filter auf zwei Dichtringen auf, von denen einer eine Öffnung in einen ebenfalls im mittleren Substrat angeordneten Mikrokanal mit anschließender Mikrokammer freigibt.

Die WO 2009/112982 A1 zeigt eine Filtrationsvorrichtung mit einem Filterelement und einer daran (z.B. in Form eines doppelseitigen Klebebands) angebrachten adhäsiven Kapillarstruktur, durch welche das gefilterte Fluid vom Filter zu einer Detektionsstelle geleitet wird. Die Kapillarstruktur ist hierbei eine mikrofluidische Struktur, die Kapillarkräfte erzeugt und deckt mindestens 25 % der unteren Oberfläche des Filterelements ab.

Die US 2007/269893 A1 zeigt ein Gerät zum Sammeln von Blut und zur Separierung von Blutplasma als Flüssigkeitsprobe, wobei ein Kanal über die Nutzung von Kapillarkräften die Flüssigkeitsprobe absorbiert. Zur gleichmäßigen Befüllung findet direkt unterhalb der Separierungsvorrichtung eine Belüftung in Querrichtung zur Hauptfüllrichtung in der Nähe des Kanals statt.

Die Erfindung findet Verwendung bei mikrofluidischen Vorrichtungen, die zur Fluidseparation, insbesondere Blutseparation eingesetzt werden.

Bei der Separation von Partikeln aus einem Fluid, wie beispielsweise einer Blutseparation, wird das zu separierende Medium, in diesem Fall Blut, auf einen Filter gegeben. Das Fluid und kleine Bestandteile durchströmen den Filter und werden über Kanäle in der Vorrichtung abtransportiert. Um bestimmte Eigenschaften des Fluides zu detektieren, wird das Fluid in Kontakt zu Reagenzien gebracht, die eine chemisch oder physikalisch detektierbare Wechselwirkung hervorrufen, z.B. eine Verfärbung des Fluides bei einer Nachweisreaktion.

Für diese verschiedenen nasschemischen, biochemischen oder diagnostischen Analysen ist es notwendig, dass sich das Fluid während eines definierten Zeitintervalls mit Reagenzien in einer Kammer oder einem Reservoir vermischt und diese dabei löst und/ oder mit diesen reagiert.

Die Reagenzien können dabei an eine Kammer, einen Kanal oder eine Reservoirwand oder auch an Partikeloberflächen gebunden sein.

Bei immunochemischen Analysen sind die Reagenzien beispielsweise Antikörper, Enzyme, biotinylierte Proteine und Antikörper, Streptavidien oder Phosphatasen.

Es ist bei verschiedenen solchen Lösungsvorgängen oder Reaktionsvorgängen erforderlich, dass ein bestimmter vorgegebener Volumenstrom oder Massestrom in einem bestimmten Zeitintervall an Fluid zur Verfügung gestellt wird, um ein sicheres Lösen des Reagenzes oder eine sichere Reaktion zu erreichen.
Bei einer Unterschreitung oder einem Abreißen des notwendigen Fluidstromes besteht beispielsweise die Gefahr, dass Bestandteile einer in der mikrofluidischen Analyseeinrichtung angeordneten getrockneten, insbesondere pulverförmigen Substanz verklumpen oder in Depots haften bleiben. Dies kann dazu führen, dass das Ergebnis einer Nachweisreaktion verfälscht wird.

Auch in Anwendungen, bei denen mehrere Kammern parallel oder sequentiell gefüllt werden sollen, ist ein ausreichender Fluidstrom zur homogenen Befüllung der Kammern erforderlich.

Kritisch für die Bereitstellung einer homogenen Fluidzufuhr in Nachweiskammern ist beispielsweise ein Luftblaseneinschluss im Separationsbereich oder im Bereich des Kanaleintrittes eines fluidabführenden Kanals, da durch eingeschlossene Luftblasen der Volumenstrom vermindert wird oder ganz abreißen kann.

Der Erfindung liegt vor diesem Hintergrund die Aufgabe zugrunde, eine vorgegebene Menge an zu untersuchendem Fluid in einem vorgegebenen Zeitintervall in einem Untersuchungsbereich bereitzustellen.

Weiterhin liegt der Erfindung die Aufgabe zugrunde eine mikrofluidische Vorrichtung konstruktiv derart auszuführen, dass eine sichere Zufuhr des separierten Fluides vom Eingabebereich zum Analysebereich erfolgt.

Da Klebeschichten und auch manche Kunststoffe, wie sie in mikrofluidischen Cartridges typisch verwendet werden, hydrophob sind, können diese einen Eintritt von wässrigen Flüssigkeiten in einen Zuführkanal verhindern oder behindern.

Vor diesem Hintergrund liegt der Erfindung die weitere Aufgabe zugrunde, die kapillare Apertur eines Fluidabführkanals konstruktiv und/oder funktionell so auszuführen, dass eine sichere Benetzung des Abführkanals erreicht wird, dass heißt, es muss gewährleistet sein, dass die separierte Flüssigkeit vom Abführkanal aufgenommen werden kann und von diesem abtransportiert werden kann.

Aus der EP 1548433 A1 ist eine Separationsvorrichtung bekannt, bei der die zu separierende Flüssigkeit durch eine Zuführöffnung auf eine Separationsmembran gegeben wird und der Filterprozess in Dickenrichtung der Membran erfolgt. Das gefilterte Fluid wird in einer Einfüllkammer aufgenommen und durch Kapillarkanäle abgeleitet.

Nachteilig an dieser Anordnung ist das hohe Totvolumen der Einfüllkammer und die Gefahr, dass in der Kammer befindliche Luft nicht homogen durch das Fluid verdrängt wird, sondern Luftblasen im Fluid bildet, die den Fluidfluss behindern.

Unter Totvolumen wird dasjenige Volumen der fluidischen Kanalstrukturen und Kammern verstanden, welches nicht als Reaktionsvolumen wirkt. Da die Einfüllkammer nach den EP 1548433 A1 zunächst vollständig gefüllt werden muss und Fluid in der Kammer verbleibt, ist das Kammervolumen der Einfüllkammer somit Totvolumen.

Die EP 1 054 805 B1 beschreibt eine Methode, um ein mikrofluidisches Netzwerk von einer zentralen Probenaufgabe, die einen kapillar aktiven Öffnungsquerschnitt besitzt, zu befüllen. Zum Abtransport der Probe von der Probenaufgabe, die in Form einer Einlasskammer gestaltet ist, mündet ein Zuführkanal in eine Wand der Kammer. Um die kapillare Probenaufnahme durch den Zuführkanal zu verbessern, wird die kapillare Apertur des Zuführkanals dadurch vergrößert, dass ein zur Einlasskammer offener umlaufender Kapillarkanal mit dem Zuführkanal fluidisch verbunden ist.
Kommt eine Flüssigkeit mit diesem kapillaren Kanal oder Spalt in Berührung, so kapillarisiert die Flüssigkeit automatisch in das mikrofluidische Netzwerk, sofern der Kontaktwinkel der Flüssigkeit mit dem Substrat und die Viskosität der Flüssigkeit nicht zu hoch sind.

Nachteilig an dieser Anordnung ist wiederum ein erhöhtes Totvolumen der Anordnung, da separierte Probenflüssigkeit vom umlaufenden Kapillarkanal zurückgehalten werden kann.

Vor diesem Hintergrund stellt sich die weitere Aufgabe der Erfindung, eine verbesserte Separationseinrichtung mit vermindertem Totvolumen, anzugeben.

Diese Aufgaben werden erfindungsgemäß gelöst durch eine mikrofluidische Vorrichtung mit den Merkmalen des Anspruchs 1.

Die erfindungsgemäße mikrofluidische Separationsvorrichtung erlaubt es, einen hinreichenden Volumenstrom an Fluid bereitzustellen. Hierzu weist die Vorrichtung ein Mittel zur Separation, insbesondere Blutfilterung auf und umfasst einen Abführkanal, der das separierte Fluid aufnimmt und ableitet. Die erfindungsgemäße Vorrichtung erlaubt es, ein mikrofluidisches Netzwerk und/oder eine Kammer aus einem Abführkanal mit geringerem aktivem Kapillarquerschnitt oder einer geringeren Öffnungsapertur zu befüllen. Zudem kann mittels der beschriebenen Methode das Totvolumen einer Zuführeinrichtung und/oder Separationseinrichtung auf ein Minimum reduziert werden.

In mikrofluidischen Cartridges wird in der Regel das mikrofluidische Netzwerk in ein plattenförmiges Substrat eingeformt. Hierzu wird bevorzugt ein formbarer Kunststoff wie Polystyrol (PS), Polymethylmethacrylat (PMMA), Polycarbonat (PC), Olefinpolymeren und Olefincopolymeren wie Cycloolefinpolymer und Cylcoolefincopolymer (COC und COP), Polyamid (PA), Polypropylen (PP), Polyethylen (PE) oder Polyethyletherketon (PEEK) in eine Negativform eingespritzt.

Die Kanalstrukturen werden einer Folie verschlossen, die auf dem Substrat angeordnet ist.

Die Folie überdeckt dabei die in eine oder in beide Seiten der Platte eingeformten Kanal- und/oder Kammerstrukturen, wodurch ein mikrofluidisches Kanalsystem mit Strukturbreiten und Strukturhöhen von einigen 10 Mikrometer bis zu einigen wenigen Millimeter gebildet wird. Die Folie deckt das plattenförmige Bauteil partiell oder vollflächig ab.

Die Folie kann mehrschichtig aufgebaut sein. Insbesondere kann die Folie ein- oder beidseitig mit einer Klebeschicht zur Befestigung am plattenförmigen Substrat versehen sein. Die Klebeschicht ist bevorzugt eine niedrig schmelzende Laminierschicht oder Siegelschicht aus Ethylen-Vinylacetat-Copolymer (EVA). Alternativ kann als Klebeschicht auch ein Acrylatkleber vorgesehen sein.

Die Oberfläche und/oder die Kanalstrukturen können vollflächig oder teilflächig einer Oberflächenbehandlung und/oder Oberflächenbeschichtung unterzogen worden sein.
Als Oberflächenbehandlung/Oberflächenaktivierung kann z.B. eine Plasmabestrahlung/ Plasmaätzung, Gammabestrahlung oder UV- Bestrahlung zur Verbesserung der Oberflächenhaftung erfolgt sein.
Als Oberflächenbeschichtungen sind beispielsweise eine Hydrophilisierung oder Hydrophobisierung der Kanalbereiche zur verbesserten Fluidleitung und/oder Fluidsteuerung wässriger Flüssigkeiten denkbar.

Alternativ kann die Folie auch eine zusätzliche Siegelschicht aufweisen, die bei einem Heißlaminierprozess auf die Oberfläche des Substrates aufgeschweißt wird.

Weiterhin kann die Folie direkt auflaminiert sein, dass heißt, es wird ohne das Aufschmelzen einer Siegelschicht durch Druck- und Hitzeeinwirkung eine stoffschlüssige Verbindung zwischen Folie und Substrat geschaffen. Das Laminieren kann auch kalt unter bevorzugter Verwendung einer Acrylatklebeschicht erfolgen.

Bevorzugt ist die Folie eben. Es kann aber auch vorgesehen sein, die Folie lokal auszuformen, um beispielsweise deformierbare Kammern zu bilden, oder einzuformen, um druck- oder vakuumgesteuerte Ventile und Mikroaktoren zu bilden.

Zusätzlich zu einer ersten Folie kann eine zweite Folie am plattenförmigen Substrat und/oder der ersten Folie angeordnet sein. Die zweite Folie kann weitere mikrofluidische Strukturen wie Kanäle, Kammern und/oder Durchbrechungen aufweisen. Bevorzugt umfasst die zweite Folie Strukturen für einen Biosensor, insbesondere Messmittel wie elektrische Kontakte und/oder elektrische Potentialflächen und/oder optische Strukturen wie optische Lichtleiter und/oder optische Spiegelflächen.

Zum Betrieb der mikrofluidischen Cartridge in beispielsweise einem Analysegerät wird eine Menge an Fluid in eine Zuführeinrichtung eingeführt. Dies kann beispielsweise ein Bluttropfen von 5 bis 50 Mikroliter Volumen sein, für lateral-flow Tests bevorzugt 5 bis 100 Mikroliter.

Die Zuführeinrichtung ist im einfachen Fall eine Einfüllöffnung. Die Zuführeinrichtung kann darüber hinaus weitere Bestandteile umfassen. Beispielsweise kann die Zuführeinrichtung ein trichterförmiger Einsatz sein, der in eine Einfüllöffnung eingesetzt wird, um die Blutaufgabe zu erleichtern und den Eingaberaum zu erweitern. Die Zuführeinrichtung kann auch eine Fingermulde umfassen, die eine Einfüllöffnung umrandet und als Auflagefläche und/oder Positionierungsfläche eines Patientenfingers bei der Blutzugabe dient.

Diese Mulde wird bevorzugt in ein plattenförmiges Deckelelement eingebracht. Das Deckelelement bildet dann die Zuführeinrichtung.

Die erfindungsgemäße Vorrichtung zur Probenseparation umfasst weiterhin eine Trenneinrichtung mit einem Mittel zum Abtrennen und/oder Separieren und/oder Filtern von Probenbestandteilen.

Das Mittel zum Abtrennen und/oder Separieren und/oder Filtern ist vorteilhaft eine Membran oder ein Filter, dem die Probenflüssigkeit zugeführt wird und wobei die Flüssigkeit die Membran und/oder Filter durchströmt und wobei Probenbestandteile vom Filter oder der Membran zurückgehalten wird.

Der Strömungsfluss durch den Filter erfolgt durch Poren und/oder Kapillaren, die ein fluidoffenes Netzwerk durch den Filter bilden. Vorteilhaft wird ein Filter aus Glasfaser, Polysulfon oder Polyethersulfon eingesetzt.

Um Blutplasma aus einer Blutprobe zu separieren, wird bevorzugt ein Filter mit einer mittleren Porengröße von ... bis ... Mikrometern eingesetzt. Vorteilhaft wird der Filter in
einem oberen plattenförmigen Substrat in der Einfüllöffnung verschweißt. Dieses Deckelelement weist bevorzugt eine Ausnehmung an der Einfüllöffnung auf, in welche der Filter, insbesondere eine Membran eingefügt werden kann. Besonders bevorzugt wird die Membran an einer Fläche der Ausnehmung, der Befestigungsfläche verschweißt.

Der Fluidstrom wird insbesondere in vertikaler Strömungsrichtung durch die Membran oder den Filter transportiert.
Diese vertikale Richtungsangabe bedeutet, dass der Fluss in etwa senkrecht zur Substratebene einer insbesondere plattenförmigen mikrofluidischen Dosiervorrichtung erfolgt.

Dabei durchströmt der Fluss eine Membran also im Wesentlichen in Dickenrichtung.

Die Membran oder der Filter ist bevorzugt in vertikaler Richtung zwischen der Einlassöffnung und einer unter der Membran/Filter liegenden Einlasskammer oder Sammelkammer angeordnet.

Die Membran oder der Filter nehmen durch die Kapillarwirkung ihrer Poren oder Kapillaren Flüssigkeit auf und halten größere Partikel, deren Größe die der Poren oder der Kapillaren überschreiten, zurück.

Dabei verschließen sich die Poren durch Agglomeration der zurückgehaltenen Partikel teilweise, so dass sich der zur Verfügung stehende Durchströmungsquerschnitt mit Andauer des fortschreitenden Separationsvorganges reduziert. Dies bedingt ein Absinken der Flussrate des Volumenstromes an Fluid in der mikrofluidischen Vorrichtung.

An der Trenneinrichtung ist eine Einlasskammer oder auch Sammelkammer angeordnet, in welche die separierte Probenflüssigkeit einfließt.

Unter Einlasskammer oder Sammelkammer wird der Raum in der fluidischen Vorrichtung zur Probenseparation verstanden, in den die separierte Probenflüssigkeit nach durchströmen der Trenneinrichtung, insbesondere nach dem Durchströmen eines Filters oder einer Membran direkt eintritt.

Die Einlasskammer oder Sammelkammer kann ein Kanal und/oder eine Kammer sein, der direkt unterhalb einer Membran angeordnet ist und nach oben hin offen ist, so dass die separierte Probenflüssigkeit vom Kanal und/oder der Kammer aufgenommen wird.

Konstruktiv bevorzugt wird die Einlasskammer oder Sammelkammer durch den Raum gebildet, der nach oben durch die Membran oder den Filter begrenzt ist.

Die untere Filter- oder Membranfläche bildet dann eine obere Trennfläche zur Einlass- oder Sammelkammer.

Der Raum der Einlasskammer wird in dieser Ausführungsform nach unten durch das plattenförmige Substrat begrenzt, dass den Boden der Sammelkammer bildet.
Die Seiten des Raumes können durch Wände gebildet sein und/oder besonders bevorzugt von einem Entlüftungsgraben umschlossen sein, wie im Weiteren ausgeführt wird.

Die Einlasskammer oder Sammelkammer kann vollständig von der Membran oder dem Filter ausgefüllt sein. In dieser konstruktiven Ausführung ist der Filter oder die Membran sowohl Teil der Trenneinrichtung als auch Teil der Einlass- oder Sammelkammer.

Von der Einlasskammer oder Sammelkammer können ein oder mehrere Entlüftungskanäle abgehen.
Weiterhin ist die Vorrichtung konstruktiv so ausgelegt, dass separierte Probenflüssigkeit von einem oder mehreren Kanälen in lateraler Richtung abgeführt werden kann.

Vorteilhaft kann die Einlasskammer oder Sammelkammer Reagenzien enthalten, die durch den Fluidstrom gelöst werden.

Ebenso kann auch die Membran mit Reagenzien getränkt oder imprägniert sein, beispielsweise Reagenzien Glycin oder Lectin, die ein Verklumpen des Fluides, insbesondere des Blutes fördern, so dass sich größere Partikelhaufen bilden, die durch die Membran zurückgehalten werden. Bei der Zugabe des Fluides findet in der so behandelten Membran eine Separierung statt bei gleichzeitigem Lösen einer ersten Reagenz, wobei das erste Reagenz die biologischen und/oder chemischen und/oder physikalischen Eigenschaften, insbesondere die Viskosität des Fluides beeinflusst.

In der Einlasskammer oder Sammelkammer kann eine zweite Reagenz angeordnet sein, die eine Nachweisreaktion in dem Fluid hervorruft. Dies kann beispielsweise eine optische Verfärbung sein.

Da die Membran oder der Filter eine hohe Eigenkapillarität aufweisen, sind vorteilhaft Mittel vorgesehen, um ein vertikales Abfließen des Volumenstromes in die angrenzende Einlass- oder Sammelkammer zu unterstützen. Hierzu weist die Einlass- oder Sammelkammer vorteilhaft einen oder mehrere Stelen und/oder Stege und/oder rampenförmige Flächen auf. Diese können bevorzugt die eine oder mehrere in vertikaler Richtung verlaufende Kerben tragen oder bilden.

Die Stelen und/oder Stege und/oder Schrägflächen sind so ausgeführt, dass die Membran auf diesen Strukturen aufliegt. Die Höhe der Strukturen entspricht dabei vorteilhaft der Tiefe der Einlass- oder Sammelkammer, wobei deren Tiefe bevorzugt 10 Mikrometer bis 1000 Mikrometer, insbesondere 50 Mikrometer bis 500 Mikrometer beträgt.

Die Kerben an den Strukturen oder die Strukturen selbst treten in fluidischen Kontakt zu der zu kontaktierenden Membran und leiten durch ihre Kapillarwirkung Fluid aus der Membran zum Kammerboden ab, so dass die Sammelkammer benetzt wird. Alternativ oder unterstützend kann vorgesehen sein, dass die Membran ausgewölbt ist, wobei die Höhe der Wölbung der Kammertiefe entspricht, so dass die Membran am Scheitel der Wölbung am Kammerboden anliegt.
Da der Scheitel der Membran einen spitzen Winkel mit dem Kammerboden bildet, treten dort hohe Kapillarkräfte bei Benetzung der Membran auf, so dass über den Spalt zwischen Membran und Kammerboden separiertes Fluid in die Sammelkammer abgeleitet wird.

Vorteilhaft ist die Einlass- oder Sammelkammer zumindest teilweise von einem Graben umgeben, der eine im Vergleich zur Kammertiefe größere Tiefe und eine Entlüftung aufweist, so dass die Luft in der Einlass- oder Sammelkammer durch das einströmende Fluid über den Graben verdrängt werden kann. Der Einfüllgraben hat bevorzugt eine Breite von mindestens 100 Mikrometern und eine Tiefe von mindestens 5 Mikrometern.

Das Volumen der Einlass- oder Sammelkammer beträgt bevorzugt 0,01; 0,02; 0,05; 0,1; 0,2; 0,5; 1; 2; 5; 10; 20; 50; 100; 200; 500; 1000 Mikroliter, wobei auch Kammervolumen gewählt werden können, die sich aus der Addition der angegebenen Werte ergeben.

Der eingangs beschriebene Entlüftungsgraben bildet einen Fluidstop, da der Fluidstrom die Grabenstufe nicht überfließen kann. Vorteilhaft umschließt der Graben die Einlass- oder Sammelkammer, in welchem der Abführkanal verläuft, vollständig bis auf den Auslassbereich der Einlass- oder Sammelkammer, so dass die Luft aus der Einlasskammer gleichmäßig verdrängt werden kann.

Im Bereich zwischen dem Kammerauslass und den Grabenenden, die aneinander grenzen, besteht die Gefahr, dass ein ungewollter Fluidabfluss in den Graben erfolgt.
Daher ist besonders vorteilhaft die Probenseparationsvorrichtung konstruktiv derart zu gestalten, dass kein Fluideintritt in den Graben erfolgt.

Dies wird einerseits dadurch erreicht, dass die Enden des Entlüftungsgrabens aufgeweitet sind. Durch die Aufweitung wird die Kapillarstufe zum Füllen des Entlüftungsgrabens vergrößert, was bedeutet, dass die Gefahr einer ungewollten Befüllung des Entlüftungsgrabens deutlich gemindert ist.

Die erfindungsgemäße Ausführung der Separationsvorrichtung sieht vor, dass der Kanal zum Abführen der separierten Probenflüssigkeit aus der Einlass- oder Sammelkammer im Boden der Sammelkammer angeordnet ist, also der Kanal durch eine Ausnehmung im Boden der Einlasskammer gebildet wird.

Unter dem Begriff Abführkanal wird dabei ein Kanal verstanden, der eine Flüssigkeit aus der Einlasskammer aufnehmen und in ein mikrofluidisches Fluidnetzwerk überführen kann.

Die Anordnung des Abführkanals im Boden der Einlasskammer hat den Vorteil, dass die separierte Probenflüssigkeit nicht zunächst vollständig die Einlasskammer füllen muss, um durch einen seitlich gelegenen Kanal abtransportiert zu werden, sondern ein direkter Abtransport der separierten Probenflüssigkeit zu Analysebereichen der mikrofluidischen Cartridge erfolgt.

Diese konstruktive Ausgestaltung vermeidet somit die Bildung von Totvolumen an Probenflüssigkeit, die für die Analyse nicht zur Verfügung stehen würde.
Dies ist insbesondere in denjenigen diagnostischen Anwendungen von Vorteil, bei denen nur eine geringe Probenmenge, wie beispielsweise ein Blutstropfen von 10 bis 50 Mikroliter entsprechend einer separierten Menge an Blutplasma von 5 bis 20 Mikroliter Volumen zur Verfügung steht.

Weiterhin wird vorteilhaft der Abführkanal zumindest dort, wo sich der Kanal in der Nähe des Entlüftungsgrabenendes erstreckt, von einer Folie fluiddicht abgedeckt, so dass eine ungewollte Fluidverbindung zwischen Kanal und Entlüftungsgraben verhindert ist. Der Abführkanal wird auch in der Einlasskammer zumindest abschnittsweise durch die Folie abgedeckt, so dass erreicht wird, dass der Einströmbereich für die separierte Flüssigkeit in der Einlass- oder Sammelkammer liegt.

Diese Kanalabdeckung ist als Zunge ausgeführt, die den Kanal fluiddicht angenähert bis zur Mitte der Einlass- oder Sammelkammer verschließt, so dass eine zentral in die Einfüllöffnung eingebrachte und durch die Membran separierte Probenflüssigkeit direkt in den zentralen Abführkanal abfließen kann.

In einer bevorzugten Ausführung der Erfindung weist der Kanal ein Mittel zur Unterstützung der kapillaren Benetzung des Kanals auf.
Dieses Mittel kann vorteilhaft eine Kerbe mit erhöhter Kapillarwirkung sein, die dem Kammerboden mit dem Boden des Abführkanals verbindet.
Als weiteres alternatives Mittel kann mindestens eine Rampe zwischen Kammerboden und Kanalboden angeordnet sein, die in gleicher Weise kapillarverbindend wirkt.

Die Erfindung wird in den folgenden Ausführungsbeispielen näher beschrieben. Dabei zeigt
- Fig. 1:: eine erste Ausführungsform einer Cartridge (21) mit einer Vorrichtung zur Probenseparation (1)
- Fig. 2:: eine zweite Ausführungsform einer Cartridge (21) mit einer Vorrichtung zur Probenseparation (1)
- Fig. 3:: eine Ansicht der Cartridge (21) nach Fig. 2 mit Deckelfolie (6)
- Fig. 4:: eine Schnittdarstellung der Probenseparation (1) einer Cartridge nach Fig. 2 und Fig. 3
- Fig. 5:: eine Schnittdarstellung der Probenseparation (1) einer Cartridge nach Fig. 1
- Fig. 6:: eine perspektivische Ansicht der Probenseparation (1) nach den Figuren 2 und 3
- Fig. 7 bis Fig. 9:: eine Darstellung eines Querschnittes durch die Probenseparation (1) nach den Fig. 2, 3 und 6 bei Zugabe einer Probenflüssigkeit

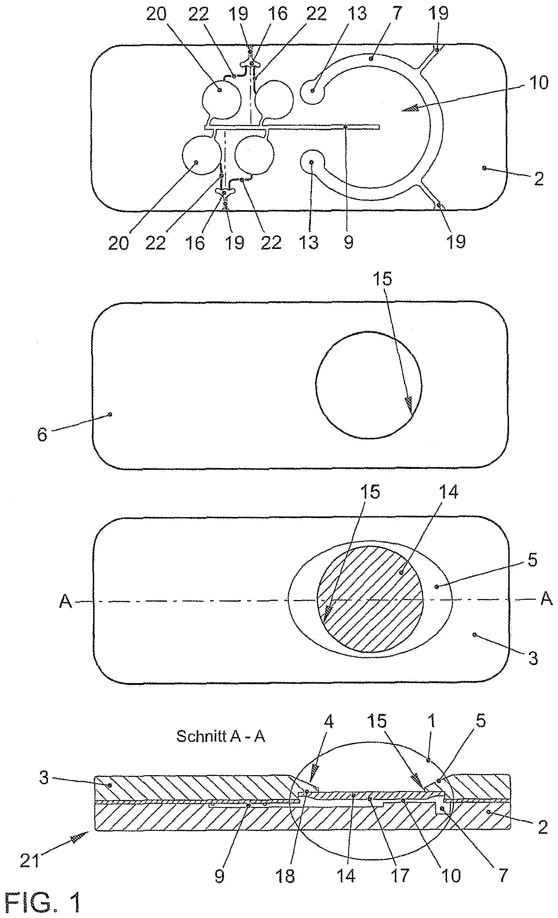

Eine Cartridge (21) mit einer erfindungsgemäßen Vorrichtung zur Probenseparation (1) ist in Figur 1 dargestellt. Hierbei wird die Cartridge (21) aus mehreren Bauteilen (2,3,6) zusammengefügt. Die Basis der Cartridge (21) bildet ein unteres plattenförmiges Substrat (2) in die mikrofluidische Strukturen mit Strukturbreiten von einigen Mikrometern bis zu einigen Millimetern eingeformt sind.

Das untere plattenförmige Substrat (2) ist insbesondere eine Kunststoffplatte und umfasst dabei einen Probenzuführbereich. Der Probenzuführbereich ist insbesondere eine Einlasskammer (10) in die eine zu separierende Probenflüssigkeit nach der Separation einfließt.

Die Einlasskammer (10) ist zumindest teilweise von einem Entlüftungsgraben (7) begrenzt. Der Entlüftungsgraben (7) besteht aus einem vorzugsweise vertieftem Kanal mit einer Breite von mindestens 100 Mikrometern und einer Tiefe von mindestens 5 Mikrometern. Der Entlüftungsgraben (7) bildet eine Kapillarstufe zur Einlasskammer (10) und wird über Entlüftungskanäle (19) entlüftet, so dass in die Einlasskammer einströmende Flüssigkeit die Luft in der Einlasskammer (10) über den Graben (7) und die Kanäle (19) verdrängen kann und wobei die separierte Probenflüssigkeit selbst am Rand des Grabens (7) stoppt.

Wie die untere schematische Zusammenbauzeichnung in Figur 1 und die Figuren 7 bis 10 zeigen, entsteht durch das Einschweißen der Membran (14) in das obere plattenförmige Substrat (3) entlang der Befestigungsfläche (18) ein umlaufender Spalt (25), der sowohl über den Zuführkanal (9) als auch über dem Entlüftungsgraben (7) verläuft.
Der durch den Membranrand erzeugte Spalt oder Ringkanal (25) verläuft koinzident über dem Entlüftungsgraben (7) und wird durch die Folie (6) am Kanalbereich überdeckt.

Die Öffnung (15) in der Folie entspricht besonders bevorzugt in etwa dem Durchmesser der Membran (14) im unbefestigten Membranbereich, so dass der Folienrand umlaufend auf der Membranfläche aufliegt. Da sehr dünne Folien von 20 Mikrometern bis 200 Mikrometern Dicke verwendet werden, die elastisch sind, wird die Folie (6) durch die Auflage auf der Membran (14) stabilisiert und verhindert einen ungewollten Fluidabfluss in den Spalt (25).

Besonders vorteilhaft ist das Ende des umlaufenden Entlüftungsgrabens (7) der Einlasskammer (10) aufgeweitet. Diese Grabenaufweitung (13) erfüllt die Aufgabe, einen Fluidabfluss von der Einlasskammer (10) oder den Abführkanal (9) in den Entlüftungsgraben (7) zu verhindern.

Die Grabenaufweitung (13) trägt dazu bei, zuverlässig zu verhindern, dass ein ungewollter Fluidfluss vom Kanal (9) über den Spalt (25) oder von der Membran (14) über den Spalt (25) in den Entlüftungsgraben (7) durch kapillare vertikale oder dreidimensionale Benetzungen erfolgt. Die Aufweitung erhöht den kapillaren Widerstand für eine ungewollte kapillare Benetzung des Entlüftungsgrabens, da der Abstand zwischen dem besagten Spalt (25) und/oder der Membran (14) zum Boden des Entlüftungsgrabens vergrößert wird.

Alternativ oder unterstützend kann vorteilhaft vorgesehen sein, dass eine hydrophob wirkende Folie (6) den Entlüftungsgraben zumindest teilweise überdeckt.

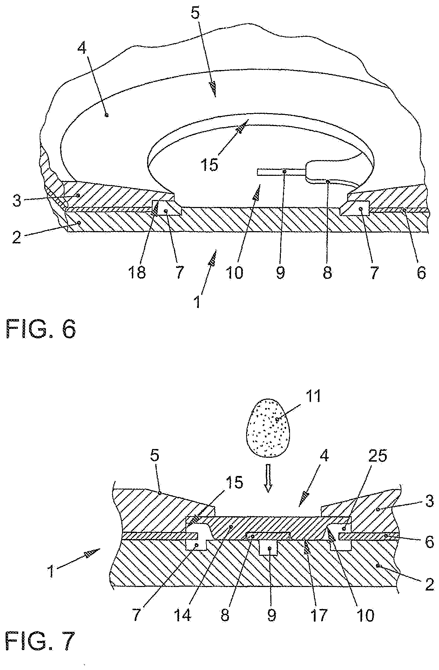

Die Figuren 7 bis 9 zeigen solch eine teilweise Überdeckung des umlaufenden Entlüftungsgrabens (7) wobei die Folie (6) etwa bis zur Mitte des Entlüftungsgrabens (7) reicht. Hierdurch wird erreicht, dass einerseits die Entlüftungsfunktion des Grabens (7) erhalten bleibt, andererseits aber keine wässrige Flüssigkeit in den Spalt (25) oder vom Spalt (25) in den Entlüftungsgraben (7) eintritt, das diese von der schwer benetzbaren hydrophoben Folie (6) zurückgedrängt wird.

Besonders vorteilhaft ist die Folie (6) so ausgeführt, dass diese den Abführkanal (9) in der Nähe der Enden des Entlüftungsgrabens (7) fluiddicht verschließt und/oder abdeckt.

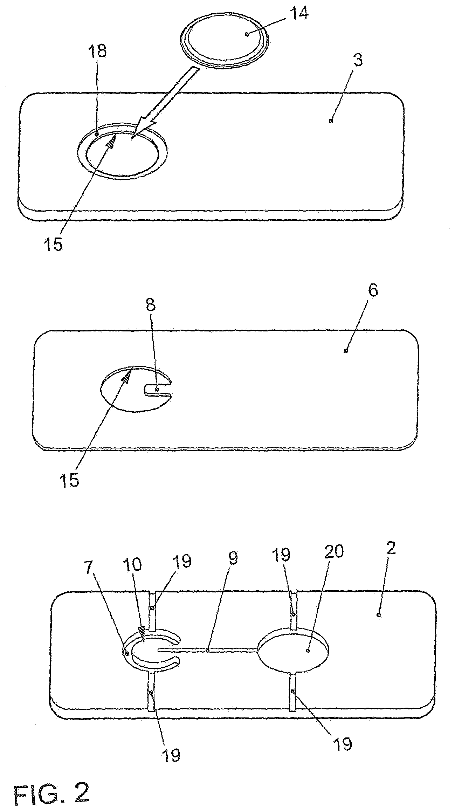

Diese Abdeckung kann eine Zunge (8) sein, die mit der Folie einstückig verbunden ist und in eine Öffnung (15) in die Folie (6) hineinragt, wie die Figuren 2, 3, 4, 6 und 7 schematisch darstellen.

Der erfindungsgemäße Aufbau nach dem ersten Ausführungsbeispiel erlaubt ein vollständiges Befüllen der Einlasskammer (10), wobei funktionell bevorzugt ist, die Einlasskammer (10) nicht vollständig zu befüllen, sondern separiertes Probenvolumen direkt mittels des Abführkanals (9) zur weiteren Reaktion und Analyse abzuführen.

Der Abführkanal (9) leitet die separierte Probenflüssigkeit in ein fluidisches Netzwerk, insbesondere kapillares Netzwerk bestehend aus Analysekammern (20) sowie weiteren Komponenten.

In einer Ausführung gemäß Fig. 1 sind mit dem Kanal (9) mindestens eine, im Ausführungsbeispiel vier Analysekammern (20) fluidisch verbunden. Hierbei können die Analysekammern (20) entweder sequentiell oder parallel befüllt werden.

So kann eine parallele oder sequentielle Befüllung durch hier nicht gezeigte aktive oder passive Ventile zur Steuerung des Flusses oder durch eine gesteuerte Entlüftung erfolgen.

Die Analysekammern (20) sind durch weitere Kanäle mit Kapillarstops (16) verbunden. Im Betrieb tritt dabei separierte Probenflüssigkeit zunächst in eine Analysekammer (20) ein, verdrängt die in der Analysekammer (20) eingeschlossene Luft, füllt die Kammer (20) vollständig luftblasenfrei und strömt dann in einen ersten Entlüftungskanal (22) ein.

Der erste Entlüftungskanal (22) wird im Weiteren mit Fluid befüllt. Durch Fluidaufnahme des ersten Entlüftungskanals (22) wird sichergestellt, dass die Analysekammer (20) vollständig befüllt wird.

Hierdurch ist also sichergestellt, dass keine Luftblasen in einer Analysekammer (20) verbleiben. Luftblasen können die diagnostische Analyse behindern oder verfälschen.

Dies kann beispielsweise dadurch geschehen, wenn in der Analysekammer Reagentien nicht gelöst werden, da Kammerbereiche unter einer Luftblase nicht benetzt werden.

Weiterhin stellt die vollständige Befüllung sicher, dass ein definiertes Probenvolumen, nämlich das Volumen einer Analysekammer (20) mit den Reagentien, insbesondere Nachweiskörpern reagiert und somit ein qualitativer wie auch quantitativer Analysewert erzielbar ist.

Da die Probenflüssigkeit in den ersten Entlüftungskanälen (22) keinen Beitrag an der Analysereaktion hat, also kein Nutzvolumen, sondern Totvolumen ist, sollte der Volumenanteil der ersten Entlüftungskanäle maximal 5% des Gesamtvolumens der Analysekammern (20) betragen.

Nach dem ersten Ausführungsbeispiel nach Figur 1 ist vorgesehen, dass jeweils zwei erste Entlüftungskanäle (22) in einen Kapillarstop (16) münden, wodurch der Fluidfluss der Probenflüssigkeit dort abbricht.

Vorteilhaft wird der Kapillarstop (16) konstruktiv dadurch gebildet, dass der Entlüftungskanal (22) eine geringere Querschnittsfläche als der Kapillarstop (16) hat.

Der Entlüftungskanal (22) und der Kapillarstop (16) teilen sich eine gemeinsame Begrenzungsfläche, nämlich die obere Begrenzungsfläche, die durch die Unterseite der Folie (6) abgegrenzt ist. Typisch ist Folienmaterial oder die Klebeschicht einer Folie hydrophob, so dass der kapillare Fluss entlang der Folienunterseite, entsprechend der Kanaloberseite (22), erschwert ist.

Bevorzugt weist der Kapillarstop (16) konstruktiv eine größere Tiefe und eine größere Breite als der erste Entlüftungskanal (22) auf.

Dadurch werden sowohl in den lateralen Richtungen als auch in einer vertikalen Richtung geometrische Kapillarstufen erzeugt.

Besonders vorteilhaft kann vorgesehen sein, dass die Folie (6) zumindest teilweise am Kapillarstop (16) Aussparungen aufweist, derart dass am Ende des ersten Entlüftungsgrabens der Kapillarstop (16) sich in die Folie (6) hinein erstreckt.

Der Kapillarstop (16) weist im Vergleich zu beiden Seitenflächen des Kanals (22) also eine größere Breite und im Vergleich zum Boden- und zur Deckenfläche des Kanals (22) auch eine größere Tiefe und eine größere Höhe auf.

Dadurch liegen in dieser besonders bevorzugten Ausführungsform sowohl in allen lateralen Richtungen als auch in allen vertikalen Richtungen Kapillarstufen vor.

Der Kapillarstop (16) wird durch einen weiteren Entlüftungsgraben (19) entlüftet, der den Kapillarstop (16) mit einer Seitenfläche der Cartridge (21) fluidisch verbindet.

Dadurch, dass die Kanalausnehmungen (9) und Kammerausnehmungen (20) durch die Folie eine Deckfläche erhalten, wird eine dreidimensionale, vollvolumige Fluidbenetzung erreicht. Um dies zu unterstützen, können die Folie (6) und/oder die Ausnehmungen (9, 22, 20) zumindest teilweise hydrophiliert sein, insbesondere in Form einer Beschichtung durch ein aufgebrachtes hydrophiles Fluid, welches eingetrocknet wird.

Die Folie (6) und/oder die Strukturen (7, 10, 13, 16, 19) können vorteilhaft auch lokal hydrophobisiert sein.

Hierbei sind der Entlüftungskanal (7) und dessen Aufweitung (13) bevorzugt vollständig hydrophibisiert, so dass die Fähigkeit eines kapillaren Benetzens des Grabens (7) und seiner Aufweitung (13) durch eine wässrige Flüssigkeit herabgesetzt ist.

Auch der Boden des Einfüllbereiches (10) kann lokal, insbesondere in der Nähe des Entlüftungsgrabens (7) hydrophibisiert sein, um ein Übertreten von wässriger Probenflüssigkeit in den Graben (7) zu vermeiden.

Insbesondere der Kapillarstop (16) wird vorteilhaft vollständig hydrophibisiert, um die kapillare Anhalte- und/oder Rückhaltefunktion des Kapillarstops zu verbessern.

Da die Folie (6) diese Strukturen abdeckt, wird diese vorteilhaft in diesen funktionellen Bereichen hydrophob beschichtet.

Dies kann beispielsweise durch lokales Bedrucken der Folie mit einer hydrophoben Beschichtung in diesen Teilbereichen erfolgen.

Das lokale Beschichten, insbesondere Spotten kann mit hydrophilen Beschichtungen ist bevorzugt in fluidleitenden Bereichen wie dem Abführkanal (9), den Analysekammern (20) und dem ersten Entlüftungskanal (22) vorgesehen sein.

Eine Beschichtung mit einem hydrophoben oder hydrophilen Film trägt funktionell in diesen Bereichen zu einer besseren und vorteilhaft vollständigen, weitgehend luftblasenfreien und weitgehend vollständigen Befüllung dieser Strukturen (9, 20, 22) bei.

Die Folie wird zumindest lokal mit einer Adhäsivschicht, insbesondere Klebeschicht versehen. Bevorzugt sind beide Folienflächen zumindest teilflächig mit einer Adhäsivschicht versehen, so dass mittels der Folie sowohl die Cartridgebasis (2) als auch das Deckelelement (3) gefügt werden.

Beim Fügeprozess wird zunächst eine erste Abdeckfolie von der Folie (6) entfernt, so dass eine erste Adhäsivschicht offen liegt. Dann werden die Cartridgebasis (2) und die Folie (6) zueinander positioniert und an der offenen Klebefläche verklebt.

Nach dem Verkleben wird eine zweite Abdeckfolie von der Folie (6) abgezogen, die beklebte Cartridgebasis (2) zum Deckelelement (3) positioniert und die Cartridgebasis (2) mit dem Deckelelement (2) über die Folie (6) verbunden.
In das Deckelelement (3) wird vorab die Membran (14) zentral eingeschweißt.
Als Produkt ergibt sich die Cartridge (21) nach der unteren Darstellung in Figur 1, die eine Separationsvorrichtung (1) nach der vorliegenden Erfindung umfasst.

Alternativ zu der verwendeten doppelseitig adhäsiven Folie kann vorgesehen sein, eine Klebeschichtfreie Folie (6) zu verwenden.
Dies kann vorteilhaft eine Folie (6) sein, die einseitig zumindest lokal eine Siegelschicht aufweist.

Bei der Cartridgeherstellung wird die Folie (6) mittels der Siegelschicht auflaminiert.
Hierzu wird die Folie mit der Siegelschicht auf der Cartridgebasis (2) positioniert, die Siegelschicht thermisch angeschmolzen und eine fluiddichte Verbindung zwischen Cartridgebasis (2) und Folie (6) hergestellt.

Alternativ kann die Folie auch insbesondere durch ein Kaltlaminierverfahren aufgebracht werden, wobei insbesondere eine Acrylatklebeschicht zum Fügen eingesetzt wird.

Bei Verwendung einer Laminierfolie (6) kann vorteilhaft vorgesehen sein das Deckelelement aufzuschweißen, über eine Nietverbindung an der Cartridgebasis zu befestigen oder eine weitere doppelseitig klebende Folie zur Befestigung des Deckelelements an der Laminierfolie vorzusehen.

Vorteilhaft weist das Deckelelement eine Mulde (5) auf. Diese unterstützt die Zugabe von Probenflüssigkeit durch ihre trichterförmige Form, die aufgegebenes Fluid im Muldenbereich aufnimmt und der Einfüllöffnung (15) zuleitet.

Besonders vorteilhaft ist die Mulde (5) angenähert bis zur Befestigungsfläche (18) eingeformt, insbesondere entspricht die Trichtertiefe der Mulde (5) in etwa dem vertikalen Abstand zwischen der Befestigungsfläche (18), der Membran (14) und der Oberfläche des Deckelementes (3).

Diese konstruktive Ausführung der Mulde (5) gewährleistet einen direkten Fluidabfluss von auf die Muldenfläche abgegebener Probenflüssigkeit in den Membranbereich.

Die Trichtertiefe beträgt vorteilhaft 0,5 Millimeter bis 10 Millimeter.

Besonders vorteilhaft ist die Mulde kreisförmig oder ellipsenförmig, wobei der Radius der langen Seite der insbesondere ellipsenförmigen Mulde (5) 1 bis 1,5 Zentimeter und der Radius der kurzen Seitenlänge 0,7 bis 1 Zenitmeter betragen soll.

Der Radius einer kreisförmigen Mulde (5) soll insbesondere 1 bis 1,5 Zentimeter betragen.

In einem weiteren zweiten Ausführungsbeispiel nach Figur 2 umfasst das untere plattenförmige Substrat (2), die Basis der Cartridge (21) ein fluidisches Netzwerk besteht aus einem Abführkanal (9) und einer durch Kanäle (13) entlüfteten Analysekammer (20).
Der Kanal (9) nimmt separiertes Probenfluid aus einer Einlasskammer (10) zentral auf.
Die Einlasskammer (10) wird durch einen umlaufenden Graben über Kanäle (19) entlüftet.

Im zweiten Ausführungsbeispiel nach Figur 2 ist das obere plattenförmige Substrat (3), das Deckelelement (3), mit der Unterseite dargestellt.

Das Deckelelement (3) weist um die Einfüllöffnung (15) eine Ausnehmung mit einer Befestigungsfläche (18) auf. In diese Ausnehmung wird eine Membran (14) zentral eingefügt, insbesondere thermisch mit der Befestigungsfläche (18) verbunden.

Die Folie (6) ist eine Laminierfolie, die auf das untere plattenförmige Substrat (2) auflaminiert wird.

Nach dem Fügen der Membran (14) in die Ausnehmung des oberen plattenförmigen Substrates (3) wird dieses Deckelelement mit dem Separationsbereich zur Öffnung (15) in der Folie positioniert und auf die Folie aufgeklebt.

Anstelle des Verklebens sind alternative Fügeprozesse wie ein Ultraschallverschweißen oder ein Vernieten einsetzbar.

Es wäre auch denkbar, die Bauteile durch ein weiteres nicht dargestelltes äußeres Gehäuse fest zueinander zu halten oder zu klemmen, beispielsweise durch Niederhalter- und/oder Positioniermittel und/oder elastischen Haltemitteln im äußeren Gehäuse.

Die Folie (6) umfasst eine Einfüllöffnung (15), in welche ein zungenförmiges Folienteil hineinragt.

In Figur 7 ist ein Querschnitt durch eine derartige gefügte Probenseparation dargestellt. Hierbei stellt die Figur 7 einen Querschnitt in Höhe der Zunge (8) dar.

Wie der Figur 7 zum zweiten Ausführungsbeispiel zu entnehmen ist, deckt die Zunge (8) den Kanal (9) im äußeren Bereich der Einlasskammer (10) ab, so dass im Außenbereich der Einlasskammer (10) kein Einströmen von Probenflüssigkeit aus der Einlasskammer (20) in den Kanal (9) erfolgt.

Im gefügten Zustand liegt die Membran (14) zumindest teilweise auf der Zunge (8) auf.

In der Ausführung nach den Figuren 7 bis 9 füllt die Membran (14) vorteilhaft vollständig aus. Die Membran liegt dabei auf der Abdeckung (8) und dem Boden der Einlasskammer (10) auf, so dass die Membran (14) die Abdeckung (8) umschließt. Besonders vorteilhaft wird dabei die Einlasskammer (10) angenähert vollständig von der Membran (14) ausgefüllt, so dass sich das Totvolumen in der Kammer Null annähert.

Unter dem Totvolumen der Einlasskammer wird dabei das Volumen in der Einlasskammer verstanden, welches zwischen der unteren Begrenzungsfläche der Membran (14), der Trennfläche (17) und dem Kammerboden der Einlasskammer liegt und eine Kapillarwirkung auf das Fluid hat. Diese Kapillarwirkung wird insbesondere durch zwischen der Membran (14) und dem Boden der Einlasskammer verbleibende Spalte erzeugt und kann Fluid in der Einlasskammer durch die Kapillarwirkung unerwünscht zurückhalten.

Auch das Volumen der Membran (14) kann zumindest teilweise durch das inhärente kapillare Netzwerk Totvolumen erzeugen, da in den Kapillarkanälen und Kapillarporen der Membran (14) Probenflüssigkeit kapillar zurückgehalten wird.

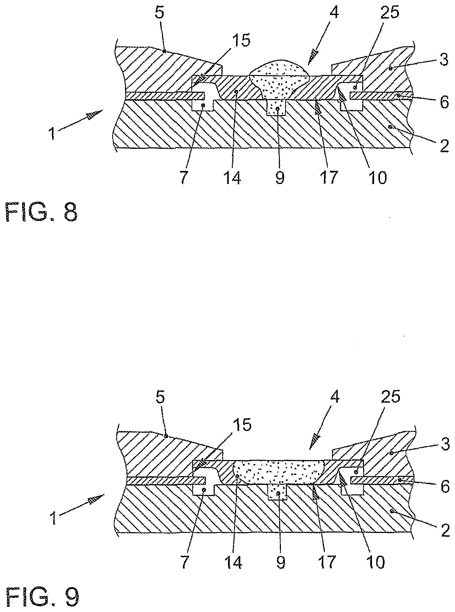

Wie die Figuren 8 und 9 schematisch darstellen, liegt die Membran (14) im Öffnungsbereich des Zentralkanals (9) bevorzugt vollflächig auf dem Boden der Einlasskammer (10) auf.

Zur Probenseparation wird eine Probe in die Zuführeinrichtung (4) eingefüllt. Nach den Figuren 7 bis 9 ist dies ein Blutstropfen.

Figur 8 und Figur 9 sind Schnitte durch die Probenseparation (1) im Zentralbereich der Einlasskammer (10).

Wie aus Figur 8 ersehen werden kann wird der Blutstropfen durch die kapillare Membran (14) aufgenommen, wobei sich größere feste Blutpartikel in der Membran abscheiden. Durch den hydrostatischen Fluiddruck der Probenflüssigkeit bildet sich in der Membran insbesondere zentral in der Einlasskammer ein Fluidtropfen aus separierter Probenflüssigkeit, hier Blutplasma.

Die Form und Länge der Abdeckung (8), nämlich die Folienzunge (8) wird derart gewählt, dass der Kanal (9) im Zentralbereich der Einlasskammer (10) nicht abgedeckt sondern nach oben hin geöffnet ist. Die obere Begrenzungsfläche des Kanals (9) wird dabei vorteilhaft durch die Unterseite der Membran (14) gebildet. Dadurch ergibt sich ein zentraler Öffnungsbereich des Kanals (9) mit geringer fluider Apertur.

Wie die Figuren 8 und 9 darstellen, kann dabei erreicht werden, dass die separierte Probenflüssigkeit, das Blutplasma direkt von dieser Kanalöffnung aufgenommen wird.

Das Plasma strömt also von der Separationsmembran (14) über die Einlasskammer (10) in die Öffnung des Kanals (9) ein, ohne die Einfüllkammer (10) während der Separation vollständig zu befüllen.

Funktionell hat das den Effekt, dass das zu analysierende Plasma direkt der Analysekammer (20) zugeführt wird.
Diese direkte Zufuhr trägt also zu einer kurzen Analysezeit für beispielsweise einen Assay bei, da fluide Befüllzeiten verkürzt sind.

Ein weiterer funktioneller Effekt der abschnittsweisen Kanalabdeckung ist das verringerte Totvolumen der Membran (14), da in dieser Anordnung ein direkter Fluidfluß in den Kanal (9) erfolgen kann, ohne dass eine vollständige Benetzung der Membran (14) stattfinden muss.

Bei verringertem Totvolumen steht somit eine höhere Menge an Probenvolumen, hier Blutplasma zur Verfügung.

Insbesondere bei Untersuchungen von Fingerprick-Blutmengen, also 1 bis 50 Mikrolitern Blut ist die Separationsmenge eine kritische Größe für diagnostische Tests, da nach der Separation of nur 5 bis zu 20 Mikroliter Plasma für beispielsweise Antikörperreaktionen zur Verfügung stehen.

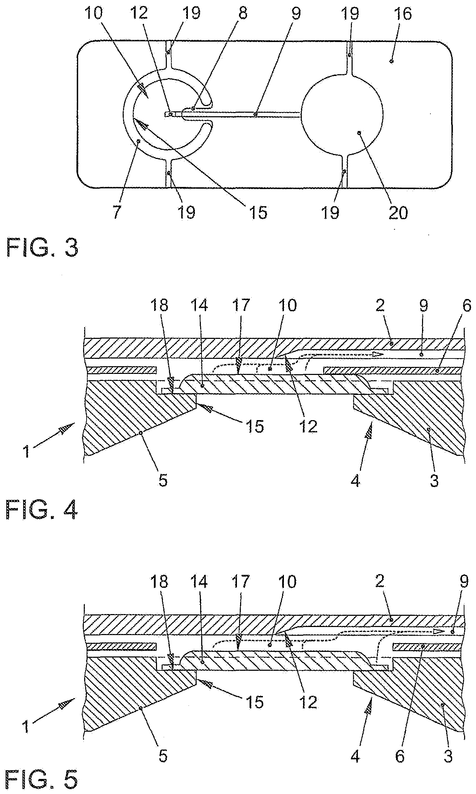

Figur 4 zeigt eine derartige Probenseparation (1) im Längsschnitt, wobei der Einfüllbereich nach unten weist.

Die Folie (6) deckt einen Abführkanal (9) zumindest abschnittsweise ab, so dass eine zentrale Öffnung des Kanals (9) verbleibt.

Besonders vorteilhaft ist der Öffnungsbereich des Kanals (9), hier das Kanalende mit einem Mittel zum Ableiten des Fluides von der Kammer in den Kanal versehen. Besonders bevorzugt ist das Mittel eine Kerbe, ein Übergangsprofil und insbesondere eine Rampe. Diese Rampe (12) vermindert den kapillaren Widerstand zwischen Kanal (9) und dem Boden der Einlasskammer (10), so dass die Benetzung des Kanals und das Einströmen von Flüssigkeit in den Kanal verbessert wird.

In Figur 4 sind die Fluidströme des Plasmas aus der Membran (10) durch die Einfüllkammer (10) in den Kanal (9) durch Pfeile symbolisiert.

Auch aus außen liegenden Bereichen der Einlasskammer kann separierte Probenflüssigkeit, im Ausführungsbeispiel Blutplasma, in den Kanal (9) transportiert werden.
Dies ist beispielsweise dann realisierbar, wenn der Kanal eine höhere Kapillarität als die Einlasskammer (10) aufweist, oder wenn das Plasma durch das Anlegen eines Überdruckes (von außen) und/oder Unterdruckes (an den Kanal (9)) durch die Probenseparation (1) in den Kanal (9) und das weitere fluidische Netzwerk befördert wird.

Figur 5 stellt eine Probenseparation nach dem ersten Ausführungsbeispiel von Figur 1 im Längsschnitt dar.

Nach diesem ersten Ausführungsbeispiel liegt der Kanal (9) innerhalb der gesamten Einfüllkammer (10) offen und nimmt in seinem Verlauf in der Einfüllkammer durchgehend Flüssigkeit auf.

Eine perspektivische Ansicht einer erfindungsgemäßen Probenseparation 1 ist in Figur 6 dargestellt. Hierbei ist die Membran (14) nicht dargestellt, um einen Einblick in die Einlasskammer (10) zu ermöglichen.

Konstruktiv entspricht diese Ausführung im Wesentlichen dem Ausführungsbeispiel nach den Figuren 2, 4 und 7 bis 9.

### Bezugszeichenliste

- 1-: Probenseparation
- 2-: unteres plattenförmiges Substrat
- 3-: oberes plattenförmiges Substrat
- 4-: Zuführeinrichtung
- 5-: Mulde
- 6-: Folie
- 7-: Entlüftungsgraben
- 8-: Kanalabdeckung
- 9-: Kanal
- 10-: Sammelkammer
- 11-: Probe
- 12-: Rampe
- 13-: Grabenaufweitung
- 14-: Membran
- 15-: Einfüllöffnung
- 16-: Kapillarstop
- 17-: Trennfläche
- 18-: Befestigungsfläche
- 19-: Entlüftungskanal
- 20-: Analysekammer
- 21-: Cartridge
- 22-: Erster Entlüftungskanal
- 25-: Spalt

## Patentansprüche

1. Vorrichtung zur Probenseparation, insbesondere zur Blutseparation, umfassend ein unteres plattenförmiges Substrat (2) mit eingeformten mikrofluidischen Kanalstrukturen und eine auf dem unteren plattenförmigen Substrat (2) angeordnete Folie (6), welche die Kanalstrukturen überdeckt,
eine Zuführeinrichtung (4) zur vertikalen Aufnahme der Probe (11),
eine Trenneinrichtung (14,15) zum Abtrennen von Probenbestandteilen, wobei die Trenneinrichtung (14, 15) eine vertikale Öffnung (15), einen Filter (14), insbesondere eine Membran (14), aufweist,
eine an der Trenneinrichtung (14,15) angeordnete und zur Trenneinrichtung (14,15) fluidoffene Einlasskammer (10) zur Aufnahme von separierter Probenflüssigkeit, insbesondere Blutplasma, und
einen Kanal (9), der die separierte Probenflüssigkeit in lateraler Richtung aus der Einlasskammer (10) abführt, wobei der Kanal (9) durch eine Ausnehmung im Boden der Einlasskammer (10) gebildet wird, so dass der Einströmbereich für die separierte Probenflüssigkeit in dem Kanal (9) in der Einlasskammer (10) liegt,
**dadurch gekennzeichnet, dass**
die Folie (6) eine Abdeckung bildet, die den Kanal (9) in der Einlasskammer (10) abschnittsweise fluiddicht abdeckt,
wobei die Abdeckung als Zunge (8) ausgeführt ist, die mit der Folie (6) einstückig verbunden ist, und wobei die Zunge (8) in eine Einfüllöffnung in der Folie (6) hineinragt, so dass der Einströmbereich des Kanals (9) im Zentralbereich der Einlasskammer (10) liegt und nicht abgedeckt, sondern nach oben hin geöffnet ist, und
wobei die untere Filterfläche eine obere Trennfläche zur Einlasskammer (10) bildet, wobei die Trennfläche (17) im Bereich der Öffnung des Kanals (9) am Boden der Einlasskammer (10) anliegt und wobei der Kanal (9) zumindest abschnittsweise unter der Trennfläche (17) verläuft, so dass durch die Trenneinrichtung separierte Probenflüssigkeit nach dem Durchströmen der Trennfläche (17) den Kanal (9) direkt benetzen und in den Kanal (9) einfließen kann.

2. Vorrichtung zur Probenseparation nach Anspruch 1, **dadurch gekennzeichnet, dass** der Einströmbereich des Kanals (9) ein Mittel zum Ableiten eines Fluides, insbesondere eine Rampe (12) aufweist, so dass ein Fluidfluss vom Kammerboden insbesondere über die Rampe (12) in den Kanal (9) erfolgt.

3. Vorrichtung zur Probenseparation nach einem der vorhergehenden Ansprüche, **dadurch gekennzeichnet, dass** die Zuführeinrichtung (4) eine Mulde (5) umfasst, die in ein oberes plattenförmiges Substrat (3) eingeformt ist.

4. Vorrichtung zur Probenseparation nach einem der Ansprüche 1 bis 2, **dadurch gekennzeichnet, dass** die Zuführeinrichtung (4) einen Einsatz umfasst, der formschlüssig an einem oberen plattenförmigen Substrat (3) angeordnet ist.

5. Vorrichtung zur Probenseparation nach einem der vorhergehenden Ansprüche, **dadurch gekennzeichnet, dass** ausgenommen eines Bereiches in welchem der Kanal (9) verläuft, die Einlasskammer von einem Entlüftungsgraben (7) umgeben ist, der einen Kapillarstop für Probenflüssigkeit (11) bildet und die Einlasskammer lateral begrenzt.

6. Vorrichtung zur Probenseparation nach Anspruch 5, **dadurch gekennzeichnet, dass** der Entlüftungsgraben an seinen Enden Grabenaufweitungen (13) aufweisen kann.

7. Vorrichtung zur Probenseparation nach einem der vorhergehenden Ansprüche, **dadurch gekennzeichnet, dass** an einem oberen plattenförmigen Substrat (3) an der Einfüllöffnung (15) eine Ausnehmung angeordnet ist, die eine Befestigungsfläche (18) für eine Membran (14) aufweist.

8. Vorrichtung zur Probenseparation nach Anspruch 1, **dadurch gekennzeichnet, dass** die Folie (6) mindestens eine Adhäsivschicht aufweist

9. Vorrichtung zur Probenseparation nach einem der Ansprüche 1 oder 8, **dadurch gekennzeichnet, dass** die Folie (6) einen Entlüftungsgraben (7) zumindest teilweise überdeckt.

## Claims

1. Device for sample separation, particularly for separating blood, comprising
a lower plate-shaped substrate (2) with channel structures moulded into the substrate (2) and a film (6) arranged on the lower plate-shaped substrate (2), wherein the film (6) covers the channel structures,
a feed device (4) for vertically receiving the sample (11),
a separating device (14,15) for separating sample constituents, wherein the separating device (14,15) comprises a vertical opening (15), a filter (14), in particular a membrane (14),
an inlet chamber (10) for receiving separated sample liquid, particularly blood plasma, said inlet chamber being arranged on the separating device (14,15) and open to fluids from the separating device (14,15), and
a channel (9) which conveys the separated sample liquid in the lateral direction from the inlet chamber (10), wherein the channel (9) is formed by a recess in the base of the inlet chamber (10), so that the inflow region for the separated liquid is located in the channel (9) in the inlet chamber (10),
**characterised in that**
the film (6) forms a cover which partly covers the channel (9) in fluidtight manner, wherein the cover is constructed as a tongue (8) which is connected in one piece to the film (6) and wherein the tongue (8) projects into a fill opening in the film (6),
so that the inflow region of the channel (9) is located in the central region of the inlet chamber (10) and is not covered but is open at the top, and
wherein the lower filter surface forms an upper separating surface towards the inlet chamber, wherein the separating surface (17) in the region of a channel opening abuts on the base of the inlet chamber (10) and wherein the channel (9) extends at least partly under the separating surface (17), so that after flowing through the separating surface (17) sample liquid separated by the separating device is able to wet the channel (9) directly and to flow into the channel (9).

2. Device for sample separation according to one of the preceding claims, **characterised in that** the inflow region of the channel (9) comprises a means for discharging a fluid, particularly a ramp (12), so that fluid flows from the base of the chamber, particularly over the ramp (12), into the channel (9).

3. Device for sample separation according to one of the preceding claims, **characterised in that** the feed device (4) comprises a well (5) which is formed in an upper plate-shaped substrate (3).

4. Device for sample separation according to one of claims 1 to 2, **characterised in that** the feed device (4) comprises an insert which is arranged in interlocking engagement on an upper plate-shaped substrate (3).

5. Device for sample separation according to one of the preceding claims, **characterised in that** apart from a region in which the channel (9) runs, the inlet chamber is surrounded by a venting trench (7) which forms a capillary stop for sample liquid (11) and laterally limits the inlet chamber.

6. Device for sample separation according to claim 5, **characterised in that** the venting trench may comprise widened portions (13) at its ends.

7. Device for sample separation according to one of the preceding claims, **characterised in that** a recess is provided on an upper plate-shaped substrate (3) at the fill opening (15), the recess comprising an attachment surface (18) for a membrane (14).

8. Device for sample separation according to claim 1, **characterised in that** the film (6) has at least one adhesive layer.

9. Device for sample separation according to one of claims 1 or 8, **characterised in that** the film (6) at least partly covers a venting trench (7).

## Revendications

1. Dispositif servant à la séparation d'échantillons, en particulier à la séparation de sang, comprenant
un substrat inférieur en forme de plaque (2) avec des structures de canaux microfluidiques pratiquées et un film (6), disposé sur le substrat inférieur en forme de plaque (2), lequel recouvre les structures de canaux,
un système d'amenée (4) servant à recevoir l'échantillon (11) de manière verticale,
un système de séparation (14, 15) servant à séparer des constituants d'échantillon, dans lequel le système de séparation (14, 15) présente une ouverture verticale (15), un filtre (14), en particulier une membrane (14),
une chambre d'entrée (10) disposée au niveau du système de séparation (14, 15) et ouverte aux fluides vers le système de séparation (14, 15) servant à recevoir un liquide d'échantillon séparé, en particulier du plasma sanguin, et
un canal (9), qui évacue le liquide d'échantillon séparé dans une direction latérale hors de la chambre d'entrée (10), dans lequel le canal (9) est formé par un évidement dans le fond de la chambre d'entrée (10) de sorte que la zone d'afflux pour le liquide d'échantillon séparé se situe dans le canal (9) dans la chambre d'entrée (10),
**caractérisé en ce que**
le film (6) forme un recouvrement, qui recouvre par endroits le canal (9) dans la chambre d'entrée (10) de manière étanche aux fluides,
dans lequel le recouvrement est réalisé sous la forme d'une languette (8), qui est reliée d'un seul tenant au film (6), dans lequel la languette (8) dépasse dans une ouverture de remplissage dans le film (6) de sorte que la zone d'afflux du canal (9) se situe dans la zone centrale de la chambre d'entrée (10), et n'est pas recouverte, mais est ouverte en direction du haut, et
dans lequel la surface filtrante inférieure forme une surface de séparation supérieure en direction de la chambre d'entrée (10), dans lequel la surface de séparation (17) repose dans la zone de l'ouverture du canal (9) au niveau du fond de la chambre d'entrée (10) et dans lequel le canal (9) s'étend au moins par endroits sous la surface de séparation (17) de sorte que le liquide d'échantillon séparé par le système de séparation humidifie directement le canal (9) après la traversée de la surface de séparation (17) et peut s'écouler dans le canal (9).

2. Dispositif servant à la séparation d'échantillons selon la revendication 1, **caractérisé en ce que** la zone d'afflux du canal (9) présente un moyen servant à dévier un fluide, en particulier une rampe (12) de sorte qu'un flux de fluide a lieu depuis le fond de chambre dans le canal (9) en passant en particulier par la rampe (12).

3. Dispositif servant à la séparation d'échantillons selon l'une quelconque des revendications précédentes, **caractérisé en ce que** le système d'amenée (4) comprend une cuvette (5), qui est pratiquée dans un substrat supérieur en forme de plaque (3).

4. Dispositif servant à la séparation d'échantillons selon l'une quelconque des revendications 1 à 2, **caractérisé en ce que** le système d'amenée (4) comprend un insert, qui est disposé par complémentarité de forme au niveau d'un substrat supérieur en forme de plaque (3).

5. Dispositif servant à la séparation d'échantillons selon l'une quelconque des revendications précédentes, **caractérisé en ce qu'**à l'exception d'une zone, dans laquelle le canal (9) s'étend, la chambre d'entrée est entourée par une tranchée d'aération (7), qui forme une butée capillaire pour du liquide d'échantillon (11) et délimite latéralement la chambre d'entrée.

6. Dispositif servant à la séparation d'échantillons selon la revendication 5, **caractérisé en ce que** la tranchée d'aération peut présenter des élargissements de tranchée (13) au niveau de ses extrémités.

7. Dispositif servant à la séparation d'échantillons selon l'une quelconque des revendications précédentes, **caractérisé en ce qu'**est disposé au niveau d'un substrat supérieur en forme de plaque (3) au niveau de l'ouverture d'aération (15) un évidement, qui présente une surface de fixation (18) pour une membrane (14) .

8. Dispositif servant à la séparation d'échantillons selon la revendication 1, **caractérisé en ce que** le film (6) présente au moins une couche adhésive.

9. Dispositif servant à la séparation d'échantillons selon l'une quelconque des revendications 1 ou 8, **caractérisé en ce que** le film (6) recouvre au moins en partie une tranchée d'aération (7).
